# EUROPÄISCHE PATENTSCHRIFT

(11) **EP 0 847 378 B1**
(45) Veröffentlichungstag und Bekanntmachung des Hinweises auf die Patenterteilung: **26.01.2000**
(21) Anmeldenummer: 96930107.6
(22) Anmeldetag: 26.08.1996
(51) Int. Cl.: C07C 29/80, C07C 41/42

(54) **VERFAHREN ZUR ABTRENNUNG VON BUTANOL UND DIBUTYLETHER MIT HILFE EINER ZWEIDRUCKDESTILLATION**
PROCESS FOR SEPARATING BUTANOL AND DIBUTYL ETHER BY MEANS OF DUAL-PRESSURE DISTILLATION
PROCEDE DE SEPARATION DU BUTANOL ET DU DIBUTYLETHER A L'AIDE D'UNE DISTILLATION A DOUBLE PRESSION

(30) Priorität: 30.08.1995 DE 19531787
(43) Veröffentlichungstag der Anmeldung: 17.06.1998
(73) Patentinhaber: BASF AKTIENGESELLSCHAFT, 67056 Ludwigshafen (DE)
(72) Erfinder: ARON, Maik, D-67251 Freinsheim (DE); RUST, Harald, D-67435 Neustadt (DE)
(86) Internationale Anmeldenummer: EP9603762
(87) Internationale Veröffentlichungsnummer: WO9708120

(56) Entgegenhaltungen:
- DE-C- 974 477
- US-A- 2 356 348
- US-A- 2 552 412
- US-A- 2 875 138
- DATABASE WPI Section Ch, Derwent Publications Ltd., London, GB; Class E17, AN 70-24101R XP002019189 & SU 245 063 A (V.Y. ARISTOVITCH, ET AL.)

## Beschreibung

Die vorliegende Erfindung betrifft ein verbessertes Verfahren zur Abtrennung von Butanol und Dibutylether aus einem Gemisch enthaltend Wasser, Dibutylether sowie n-Butanol, 2-Butanol und/oder iso-Butanol.

Butanole finden in der chemischen Industrie vielfältig Anwendung, als typische Beispiele können die Verwendung als Lösungsmittel in Lacken sowie als Einsatzstoff für die Synthese von Weichmachern genannt werden.

Die großtechnische Herstellung von Butanol erfolgt vorwiegend durch Hydroformylierung von Propen und anschließende Hydrierung zum Butanol und wird beispielsweise in D1 (Chem. Ing. Techn., 41. Jg., 1969, Seite 974-980, Dr. Dümbgen, Dr. Neubauer, "Großtechnische Herstellung von Oxo-Alkoholen aus Propylen in der BASF") erläutert. Bei dem hier beschriebenen Verfahren wird vorgeschlagen, Butanol aus Mischungen, welche Dibutylether und Wasser enthalten, durch Extraktion mit Wasser abzutrennen und anschließend eine destillative Trennung von Butanol und Wasser durchzuführen (Seite 978, zweiter Abschnitt). Dieses Trennverfahren erweist sich jedoch bei größeren abzutrennenden Mengen als relativ aufwendig, da für die Extraktion große Mengen an Wasser als Extraktionsmittel benötigt werden, welche im Anschluß wieder destillativ abzutrennen sind.

In der noch unveröffentlichten deutschen Patentanmeldung D2 (O.Z. 0050/44548, Aktenzeichen P 4400837.6) wird ein Herstellverfahren von n-Butyraldehyd und/oder n-Butanol ausgehend von 1,3-Butadien beschrieben. Die Butanolherstellung erfolgt hierbei letztlich in der Stufe d) des Verfahrens, wobei man einen Enolether in Gegenwart von Wasser und Wasserstoff umsetzt (Seite 24, Zeile 8 bis Zeile 34). Hierbei fällt ein Gemisch an, welches neben Butanol auch Dibutylether und Wasser enthält und das vor einer Weiterverarbeitung aufzutrennen ist.

Es stellte sich somit die Aufgabe, ein verbessertes Verfahren zu finden, welches verfahrenstechnisch einfach und wirtschaftlich die Abtrennung von Butanol und Dibutylether aus Mischungen enthaltend Butanol, Dibutylether und Wasser ermöglicht. Der vorliegenden Erfindung lag weiterhin die Aufgabe zugrunde, eine Abtrennung von Butanol bzw. Dibutylether in hoher Reinheit zu ermöglichen, wobei die Zugabe von zusätzlichem Wasser vermieden werden sollte.

Demgemäß wurde ein Verfahren zur Abtrennung von Butanol und Dibutylether aus einem Gemisch enthaltend Wasser, Dibutylether sowie n-Butanol, 2-Butanol und/oder iso-Butanol gefunden, welches dadurch gekennzeichnet ist, daß man
a) das Gemisch in eine erste Destillationskolonne einführt, am Sumpf dieser Destillationskolonne im wesentlichen Butanol abtrennt und daß man das am Kopf der Destillationskolonne abgezogene Gemisch
b) in eine zweite Destillationskolonne einführt und am Sumpf dieser zweiten Destillationskolonne im wesentlichen Dibutylether abtrennt und das am Kopf der zweiten Destillationskolonne gebildete Gemisch abführt, wobei man
c) die zweite Destillationskolonne bei einem höheren Druck als die erste Destillationskolonne betreibt und wobei man mindestens eines der beiden über Kopf der Destillationskolonnen abgezogenen Gemische einer Phasentrennung zuführt, wobei man im Fall der Phasentrennung hinter der ersten Destillationskolonne lediglich die abgetrennte organische Phase der zweiten Destillationskolonne zuführt und im Fall einer Phasentrennung hinter der zweiten Destillationskolonne ebenfalls eine Auftrennung in eine waßrige und eine organische Phase erfolgt und wobei man einen Teilstrom vom Kopf oder Verstärkungsteil der zweiten Destillationskolonne in die erste Destillationskolonne zurückführt.

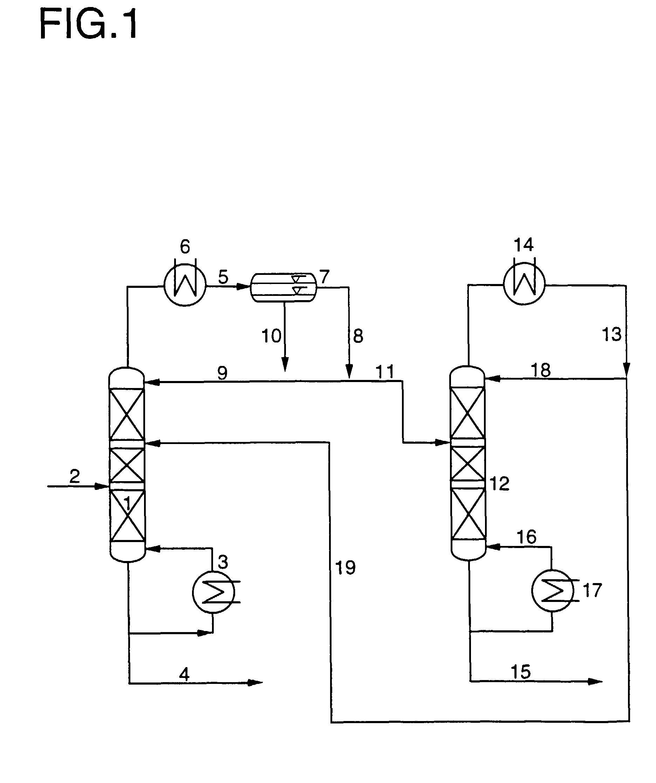

Im folgenden wird das erfindungsgemäße Verfahren anhand von Figur 1 exemplarisch näher beschrieben.

Einer Destillationskolonne (1) wird mittels Leitung (2) ein Gemisch zugeführt, welches im wesentlichen Butanol, Dibutylether und Wasser enthält. Für das erfindungsgemäße Verfahren eignen sich insbesondere Gemische, welche etwa 20 bis 99 Gew.-%, bevorzugt 70 bis 95 Gew.-% Butanol, 0,05 bis 50 Gew.-%, bevorzugt 0,5 bis 10 Gew.-% Dibutylether und 0,05 bis 50 Gew.-%, bevorzugt 1 bis 20 Gew.-% Wasser enthalten. Am Sumpf der Kolonne (1), welcher in üblicher Art und Weise mit einem Verdampfer (3) verbunden ist, wird mittels Leitung (4) nahezu reines Butanol abgezogen. Üblicherweise liegen die Konzentrationen an Butanol bei etwa 80 bis 100 Gew.-%, bevorzugt 95 bis 99,99 Gew.-%. Die restlichen Bestandteile setzen sich im wesentlichen aus Spuren von Dibutylether und Resten von Wasser zusammen.

Die erste Destillationskolonne wird üblicherweise bei einem Druck am Kolonnenkopf von etwa 0,02 bis 2 bar, bevorzugt 0,1 bis 0,7 bar betrieben. Die Temperaturen in der Kolonne liegen bei etwa 40 bis 100°C am Kolonnenkopf und etwa 65 bis 110°C am Kolonnensumpf. Für die Trennung eignen sich allgemein übliche Kolonnen. Es sind übliche Einbauten wie handelsübliche Packungen oder Böden verwendbar.

Die Größe der Kolonne richtet sich nach den jeweiligen Durchsätzen. Die theoretische Stufenzahl der Kolonne beträgt etwa 10 bis 70 Stufen, bevorzugt 15 bis 50, besonders bevorzugt 25 bis 40.

Am Kopf der Kolonne wird über Leitung (5) und Kondensator (6) ein Produktstrom abgezogen, welcher in Abhängigkeit von der Fahrweise der Kolonne vorteilhafterweise annähernd die azeotrope Konzentration aufweist.

Es ist aus der Literatur bekannt, daß Gemische aus Butanol, Wasser und Dibutylether ternäre Azeotrope bilden. So finden sich beispielsweise in D3 (Advances in Chemistry Series Nr. 116, "Azeotropic Data III", 1973, Seiten 468 bis 473 ISBN 8412-0166-8) hierzu genauere Daten. Für einige Stoffgemische sind die azeotropen Konzentrationen bei vorgegebenem Druck und Temperatur in der folgenden Tabelle dargestellt.

| Wasser | Di-n-butylether | n-Butanol | 2-Butanol | Temperatur | Druck |
|---|---|---|---|---|---|
| (Gew.-%) | (Gew.-%) | (Gew.-%) | (Gew.-%) | (°C) | (mbar) |
| 29,9 | 35,5 | 34,6 | | 90,6 | 1000 |
| 31,2 | 44,2 | 24,6 | | 45 | 130 |
| 24,7 | 19,2 | | 56,1 | 86,6 | 1000 |

Die Zusammensetzung des über Kopf abgezogenen Produktstromes wird damit nachhaltig von den vorliegenden Temperaturen und Drücken sowie von der Art der enthaltenen Komponenten - so ist es beispielsweise von Bedeutung, ob n-Butanol oder 2-Butanol enthalten ist - beeinflußt. Weiterhin beeinflußt die Fahrweise der Kolonne, wie beispielsweise der Energieeintrag, die Zusammensetzung. Daher kann die Zusammensetzung, welche annähernd die azeotrope Konzentration aufweist, nicht allgemein genau quantifiziert werden.

Der am Kolonnenkopf abgezogene Produktstrom wird einem Phasentrenngefäß (7) zugeführt, wobei das Gemisch in zwei Phasen zerfällt. Zur Phasentrennung können die in der Technik üblichen Apparate wie z.B. Schwerkraftabscheider, Zentrifugen oder Hydrozyklone, bevorzugt Dekanter, eingesetzt werden. Ein Teil der abgetrennten, vorwiegend organischen Phase wird über Leitungen (8) und (9) der ersten Kolonne als Kolonnenrücklauf zurückgeführt. Das Rücklaufverhältnis beträgt etwa 0,5 bis 20, bevorzugt etwa 1 bis 10. Die abgeschiedene, vorwiegend wäßrige Phase wird über Leitung (10) abgeführt und der restliche Teil der organischen Phase wird über Leitung (11) einer zweiten Destillationskolonne (12) zugeführt.

Die abgechiedene wäßrige Phase enthält im wesentlichen Wasser, Anteile von Butanol und Spuren von Dibutylether. Die Konzentrationen der einzelnen Komponenten hängen stark von den tatsächlich vorliegenden Bestandteilen ab. Der Gehalt an Butanol in der wäßrigen Phase schwankt etwa zwischen 4 bis 20 Gew.-%, der Anteil an Dibutylether beträgt maximal etwa 0,1 Gew.-%.

Die zweite Destillationskolonne wird bei einem höheren Druck als die erste Destillationskolonne betrieben. Der Differenzdruck zwischen den beiden Kolonnen beträgt etwa 0,05 bis 5 bar, bevorzugt 0,2 bis 2 bar, besonders bevorzugt 0,5 bis 1 bar.

Die Temperaturen in der Kolonne liegen bei etwa 60 bis 110°C am Kolonnenkopf und etwa 120 bis 170°C am Kolonnensumpf. Für die Trennung eignen sich allgemein übliche Kolonnen. Es sind übliche Einbauten wie Packungen oder Böden verwendbar.

Die Größe der Kolonne richtet sich nach den jeweiligen Durchsätzen. Die theoretische Stufenzahl der Kolonne beträgt etwa 5 bis 50, bevorzugt 10 bis 35, besonders bevorzugt 15 bis 25.

Auch in der zweiten Kolonne wird ein Produktstrom, welcher vorteilhafterweise eine annähernd azeotrope Konzentration aufweist, am Kopf über Leitung (13) und Kondensator (14) abgezogen. Das jetzt vorliegende Azeotrop weist jedoch aufgrund der Druckerhöhung eine niedrigere Konzentration an Dibutylether auf, sodaß am Sumpf der Kolonne über Leitung (15) ein Produktstrom abgezogen wird, der im wesentlichen Dibutylether enthält. Weitere Bestandteile in diesem Strom sind Butanol und Spuren von Wasser.

Die Konzentration an Dibutylether beträgt etwa 40 bis 100 Gew.-%, bevorzugt 95 bis 100 Gew.-% und kann über den Energieeintrag in die Kolonne beeinflußt werden.

Die zweite Kolonne ist in an sich bekannter Weise über Leitung (16) mit einem Verdampfer (17) verbunden.

Der am Kopf der zweiten Kolonne abgezogene Produktstrom wird teilweise über Leitung (18) in die Kolonne zurückgeführt. Das Rücklaufverhältnis beträgt etwa 0,5 bis 10, bevorzugt 0,6 bis 3.

Der übrige Teil des Produktstromes wird über Leitung (19) in die erste Kolonne zurückgeführt. Diese Rückführung kann sowohl an den Kolonnenzulauf über Leitung (2) als auch separat in die Kolonne erfolgen. Bevorzugt leitet man den Produktstrom in die Kolonne (1) zwischen dem Zulauf (2) und dem Rücklauf (9) ein. Der optimale Ort des Zulaufs ist abhängig von der Zusammensetzung des Zulaufstromes (2) und der Fahrweise der Kolonnen (1) und (12) und kann von dem Fachmann durch Routineversuche ermittelt werden.

Insbesondere falls das frisch in die Destillationskolonne (1) aufgegebene Gemisch relativ wenig Wasser enthält, kann es sich empfehlen, auch Teile der mittels des Phasentrenngefäßes abgeschiedenen wäßrigen Phase wieder in die erste Kolonne zurückzuführen. Das so zurückgeführte Wasser kann zusätzlich als Schleppmittel für den Dibutylether wirken. Die genaue Anordnung der Rückführung sowie die Menge der rückgeführten wäßrigen Phase hängen stark von dem jeweiligen Einzelfall und den vorliegenden Konzentrationen ab. Es kann sich sowohl ein Rücklauf am Kolonnenkopf als auch unterhalb des Kolonnenkopfes, im Verstärkungsteil oder in der oberen Hälfte des Abtriebteils besonders empfehlen.

Falls in dem frischen Zulaufgemisch neben Butanol, Wasser und Dibutylether weitere relativ schwer siedende Komponenten enthalten sind, kann die Auftrennung ähnlich der zuvor beschriebenen Verfahrensweise erfolgen. Die zusätzlich vorhandenen Komponenten werden praktisch vollständig im Sumpf der ersten Kolonne zusammen mit dem Butanol ausgetragen und können anschließend - beispielsweise durch Destillation - abgetrennt werden. Als Beispiele für solche Komponenten können 1-Octanol oder iso-Decanol aufgeführt werden. Das erfindungsgemäße Verfahren kann auch bei hohen Konzentrationen dieses Komponenten angewendet werden. Vorteilhafterweise erstrecken sich die Konzentrationen von etwa 0 bis 20 Gew.-%.

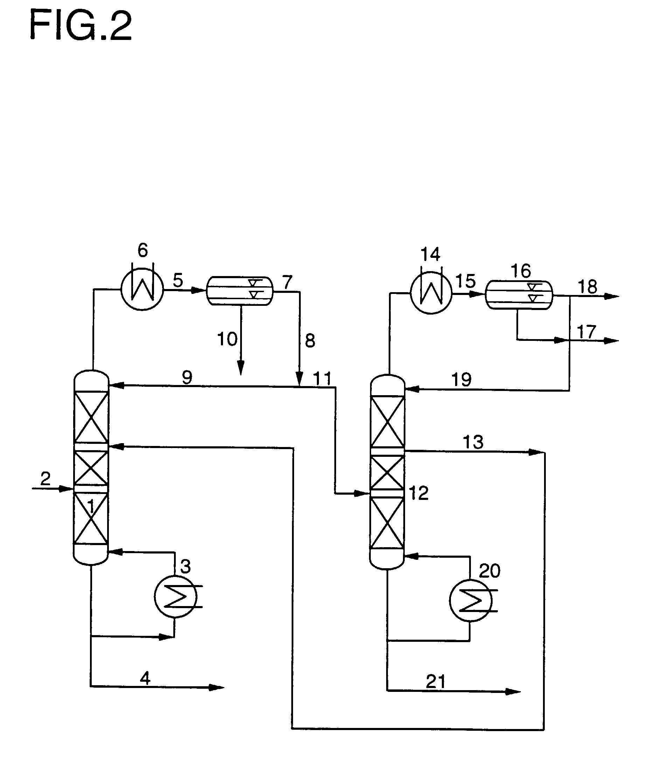

Ist in dem Zulaufgemisch ein zusätzlicher Stoff enthalten, dessen Siedepunkt niedriger als der Siedepunkt des ternären Azeotropes liegt, oder ist im Zulaufgemisch ein Stoff enthalten, der mit den oben genannten Stoffen ein binäres, ternäres oder quarternäres Azeotrop bildet, dessen Siedepunkt niedriger als der Siedepunkt des oben genannten ternären Azeotropes liegt, so kann auch diese Trennaufgabe mit dem erfindungsgemäßen Verfahren gelöst werden. In Figur 2 wird eine für diesen Fall geeignete Ausführungsform, welche in vielen Punkten mit der in Fig. 1 dargestellten Ausführungsform übereinstimmt, gezeigt.

Im folgenden wird die in Fig. 2 veranschaulichte Ausführungsform näher erläutert. Bezüglich dieser zweiten Ausführungsform wird-falls im folgenden nicht als Unterschied zu der in Fig. 1 beschriebenen Ausführungsform erläutert - auf die voranstehend gemachten Erläuterungen Bezug genommen.

Der Destillationskolonne (1) wird über Leitung (2) ein frisches Zulaufgemisch zugeführt, welches neben den Komponenten Butanol, Dibutylether und Wasser weiterhin eine relativ leichtsiedende Komponente, im folgenden Leichtsieder genannt, enthält. Der Sumpf der Kolonne ist mit einem Verdampfer (3) verbunden und über Leitung (4) wird nahezu reines Butanol abgezogen.

Am Kopf der Kolonne wird über Leitung (5) und Kondensator (6) ein Produktstrom abgezogen, welcher neben dem nahezu azeotropen Gemisch aus Butanol, Dibutylether und Wasser auch den Leichtsieder enthält.

Dem Phasentrenngefäß (7) wird über Leitung (8) die organische Phase entnommen und über die Leitungen (9) und (11) teilweise in die erste Kolonne zurückgeführt bzw. der zweiten Destillationskolonne (12) zugeführt. Die wäßrige Phase wird über Leitung (10) vom Phasentrenngefäß abgeführt. Die in dem Gemisch enthaltenen Leichtsieder reichern sich hierbei üblicherweise insbesondere in der organischen Phase an. Die genauen Konzentrationsverhältnisse hängen stark von den jeweiligen Betriebsbedingungen und der Art der eingesetzten Stoffe ab und können daher nicht allgemein quantifiziert werden.

Der zweiten Destillationskolonne, welche gemäß den vorstehenden Ausführungen bei einem höheren Druck als die erste Kolonne betrieben wird, entnimmt man über Leitung (13) einen Seitenstrom, , welcher neben dem annähernd azeotropen Gemisch auch Anteile des Leichtsieders enthält. Die Entnahmestelle befindet sich bevorzugt im Verstärkungsteil der Kolonne, besonders bevorzugt in der oberen Hälfte des Verstärkungsteils.

Der Anteil des in diesem Seitenstrom enthaltenen Leichtsieders kann aufgrund der vielschichtigen Einflüsse nicht allgemein genau quantifiziert werden, die Konzeption ist jedoch so vorzunehmen, daß man am Kopf der zweiten Kolonne über Leitung (15) und Kondensator (14) überwiegend Leichtsieder mit relativ geringen Anteilen an Wasser, Butanol und Dibutylether abführt. Der Anteil des Leichtsieders sollte etwa 30 bis 99 Gew.-%, bevorzugt 80 bis 99 Gew.-% betragen.

Dem nachgeschalteten Phasentrenngefäß (16) wird über Leitungen (17) und (18) eine vorwiegend wäßrige bzw. vorwiegend Leichtsieder enthaltende Phase entnommen.

Über Leitung (19) wird ein Teil der vorwiegend Leichtsieder enthaltenden Phase in die Kolonnne zurückgeführt.

Der Sumpf der Kolonne ist mit einem Verdampfer (20) verbunden und über Leitung (21) wird Dibutylether von hoher Reinheit abgezogen.

Der über Leitung (13) entnommene Seitenstrom wird in die erste Kolonne zurückgeführt. Die Zurückführung erfolgt gemäß dem ersten Ausführungsbeispiel.

Als Leichtsieder kann beispielsweise n-Butyraldehyd aufgeführt werden. Die Anteile des Leichtsieders im Zulaufgemisch erstrecken sich üblicherweise von etwa 0,01 bis 30 Gew.-%, bevorzugt 0,1 bis 2 Gew.-%.

Für die gemäß Fig. 2 beschriebene Ausführungsform wird ansonsten auf die gemäß Fig. 1 beschriebene Variante Bezug genommen, d.h. die hier gemachten Angaben zu Drücken, Geometrien, Konzentrationen etc. können im wesentlichen auf die zweite Variante übertragen werden.

Ob weitere Phasentrenngefäße beim erfindungsgemäßen Verfahren eingesetzt werden und welche Stellen hierfür ausgewählt werden, hängt davon ab, inwieweit Zweiphasigkeit auftritt und eine Abtrennung erwünscht ist. So kann sich beispielsweise ein Phasentrenngefäß in Strom 13 gemäß Figur 2 empfehlen.

Das erfindungsgemäße Verfahren eignet sich zur Abtrennung von Butanol und Dibutylether aus Gemischen enthaltend Wasser, Dibutylether und Butanol. Besonders eignet es sich zur Abtrennung von n-Butanol, es können jedoch auch 2-Butanol oder iso-Butanol sowie Mischungen enthaltend n-Butanol und iso-Butanol oder n-Butanol und 2-Butanol aufbereitet werden.

Falls Mischungen aufzutrennen sind, welche n-Butanol und iso-Butanol bzw. n-Butanol und 2-Butanol enthalten, wird n-Butanol im wesentlichen am Sumpf der ersten Kolonne abgeschieden. In diesem Fall ist 2-Butanol bzw. iso-Butanol als ein Zusatzstoff zu verstehen, der mit Dibutylether und Wasser ein leichtersiedendes Azeotrop als das Gemisch n-Butanol, Dibutylether und Wasser bildet. Auch in diesem Fall kann das erfindungsgemäße Verfahren gemäß der Variante von Fig. 2 angewendet werden. In diesem Fall wird das leichtersiedende Azeotrop am Kopf der Kolonne (12) als Strom (15) gewonnen.

Das erfindungsgemäße Verfahren bietet die Möglichkeit, Butanol und Dibutylether verfahrenstechnisch einfach und wirtschaftlich aus Mischungen enthaltend Butanol, Wasser und Dibutylether abzutrennen. Es können so Butanol mit einer Reinheit größer etwa 95 Gew.-%, bevorzugt 99,9 Gew.-% und Dibutylether mit einer Reinheit größer 85 Gew.-%, bevorzugt 98 Gew.-% erzielt werden. Vorteilhafterweise kann bei dem erfindungsgemäßen Verfahren die Zugabe von zusätzlichem Wasser, welches später wieder destillativ abgetrennt werden muß, vermieden werden.

### Beispiele

### Beispiel 1

Es wurde ein Versuchsaufbau gemäß Fig. 1 gewählt. Die erste Kolonne mit einem Durchmesser von 50 mm verfügt über 60 Glockenböden. Bei einem Rücklaufverhältnis der organischen Phase von 3 ergibt sich mit einem Kopfdruck von 250 mbar eine Sumpftemperatur von 86°C und eine Kopftemperatur von 71°C. Der Zulauf des Rohgemisches erfolgt auf dem 40. und der Zulauf des Rückstromes auf dem 50. Boden. Die zweite Kolonne wird mit einem Kopfdruck von 1 bar betrieben. Bei einem Rücklaufverhältnis von 3 stellen sich 146°C im Sumpf und 106°C im Kopf ein. Die zweite Kolonne hatte einen Durchmesser von 43 mm. Die Kolonne verfügt über 22 theoretische Trennstufen und ist mit einer strukturierten Packung ausgestattet. Es wurde eine Gewebepackung des Typs CY der Firma Sulzer verwendet. Der Zulauf erfolgt auf Stufe 8. Die Zusammensetzung der einzelnen Ströme ist folgender Tabelle zu entnehmen:

| | Strom 2 | Strom 4 | Strom 11 | Strom 10 | Strom 15 | Strom 19 |
|---|---|---|---|---|---|---|
| Massenstrom (kg/h) | 0,50 | 0,435 | 0,595 | 0,05 | 0,015 | 0,58 |
| n-Butanol (Gew.-%) | 87,0 | 99,979 | 61,265 | 5,22 | 0,109 | 63,189 |
| Di-n-butyl-ether (Gew.-%) | 3,0 | 0,001 | 29,769 | 0,0 | 99,891 | 27,588 |
| Wasser (Gew.-%) | 10,0 | 0,02 | 8,966 | 94,78 | 0,0 | 9,223 |

### Beispiel 2

Es wurde ein Versuchsaufbau gemaß Fig. 2 gewählt, allerdings war der zweite Kolonne kein Phasentrenngefäß nachgeschaltet. Als Leichtsieder ist im Zulauf 1 Gew.-% Butyraldehyd enthalten. Die Kolonnendaten entsprechen denen aus dem 1. Beispiel. Der Seitenabzug der 2. Kolonne erfolgt von der 19. theoretischen Stufe. Bei 250 mbar Kopfdruck in der ersten Kolonne ergibt sich eine Sumpf- bzw. Kopftemperatur von 87°C bzw. 71°C. Die Sumpf und Kopftemperatur der zweite Kolonne beträgt bei einem Kopfdruck von 1 bar 146°C bzw. 80°C. Die Zusammensetzung der einzelnen Ströme ist folgender Tabelle zu entnehmen:

| | Strom 2 | Strom 4 | Strom 11 | Strom 10 | Strom 13 | Strom 21 | Strom 18 |
|---|---|---|---|---|---|---|---|
| Massenstrom (kg/h) | 0,50 | 0,43 | 0,59 | 0,05 | 0,57 | 0,015 | 0,005 |
| n-Butanol (Gew.-%) | 86,0 | 99,94 | 58,49 | 5,14 | 59,97 | 0,11 | 20,30 |
| Di-n-butyl-ether (Gew.-%) | 3,0 | 0,006 | 30,19 | 0,0 | 28,57 | 99,87 | 25,85 |
| Wasser (Gew.-%) | 10,0 | 0,0 | 8,30 | 94,63 | 8,76 | 0,0 | 5,68 |
| Butyraldehyd (Gew.-%) | 1,0 | 0,054 | 3,02 | 0,23 | 2,70 | 0,02 | 48,17 |

### Beispiel 3

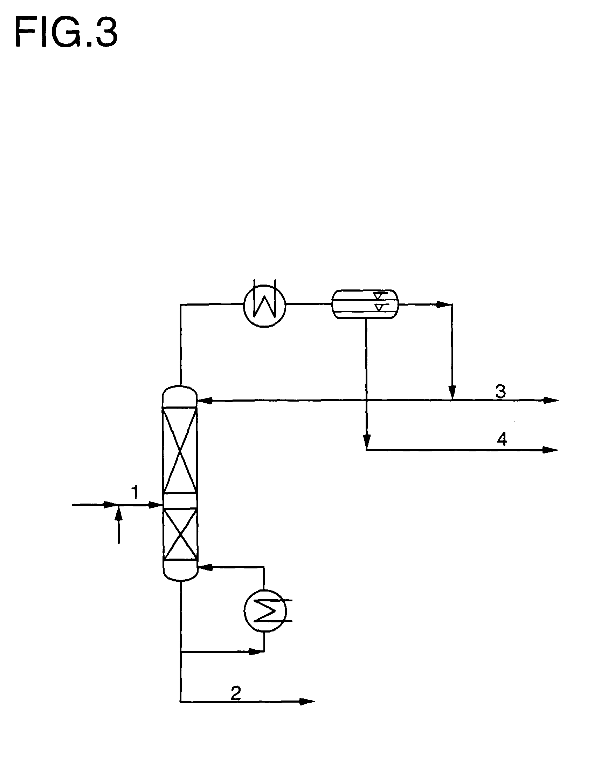

In einer Laborkolonne (Durchmesser 50 mm, Füllung mit 5 mm Netzdrahtwendeln, ges. Füllhöhe 1,8 m) wird ein Gemisch aus iso-Butanol, Di-n-butylether und Wasser destilliert. Der Zulauf erfolgt in einer Höhe von 1,3 m, gerechnet ab Beginn der Schüttung oberhalb des Sumpfes. Der Zulauf in die Kolonne setzt sich aus einem frischen Zulauf sowie aus einem Teilstrom einer zuvor durchgeführten Destillation zusammen. Die Anordnung gibt Fig. 3 wieder. Das Rücklaufverhältnis der organischen Phase beträgt 5, der Kopfdruck 250 mbar. Die Zusammensetzung der Ströme ist folgender Tabelle zu entnehmen:

| | Strom 1 | Strom 2 | Strom 3 | Strom 4 |
|---|---|---|---|---|
| Massenstrom (kg/h) | 505 | 230 | 262 | 14 |
| iso-Butanol (Gew.-%) | 87 | 99,58 | 80,1 | 8 |
| Di-n-butyl-ether (Gew.-%) | 3 | 0,4 | 5,8 | 0 |
| Wasser (Gew.-%) | 10 | 0,02 | 14,1 | 92 |

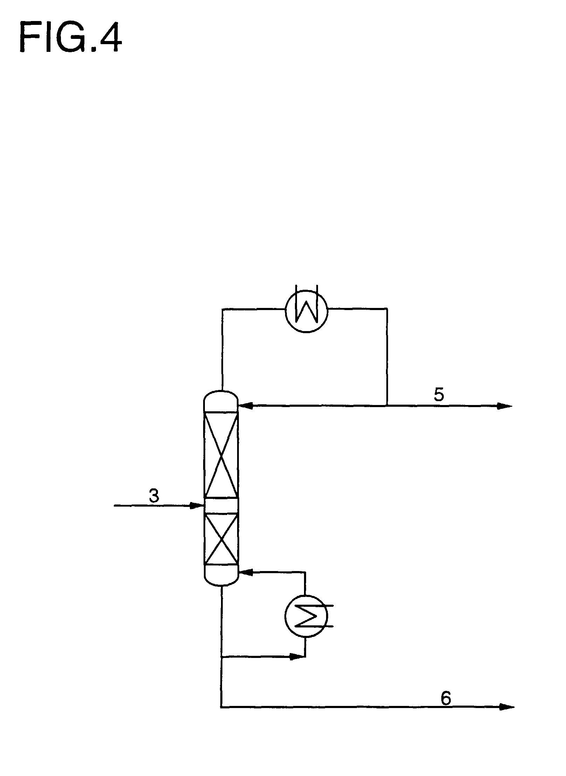

In der gleichen Kolonne wird anschließend gemäß Fig. 4 ein Gemisch entsprechend der Zusammensetzung des Stromes 3 bei 1 bar destilliert. Das Rücklaufverhältnis beträgt 3. Der Zulauf erfolgt in einer Höhe von 0,5 m, gerechnet ab Beginn der Schüttung oberhalb des Sumpfes.

| | Strom 3 | Strom 5 | Strom 6 |
|---|---|---|---|
| Massenstrom (kg/h) | 262 | 249 | 14,5 |
| iso-Butanol (Gew.-%) | 80,1 | 85,14 | 4,87 |
| Di-n-butyl-ether (Gew.-%) | 5,9 | 0,63 | 95,07 |
| Wasser (Gew.-%) | 14 | 14,23 | 0,06 |

## Patentansprüche

1. Verfahren zur Abtrennung von Butanol und Dibutylether aus einem Gemisch enthaltend Wasser, Dibutylether sowie n-Butanol, 2-Butanol und/oder iso-Butanol, dadurch gekennzeichnet, daß man
a) das Gemisch in eine erste Destillationskolonne (1) einführt, am Sumpf dieser Destillationskolonne im wesentlichen Butanol abtrennt und daß man das am Kopf der Destillationskolonne abgezogene Gemisch
b) in eine zweite Destillationskolonne (12) einführt und am Sumpf dieser zweiten Destillationskolonne im wesentlichen Dibutylether abtrennt und das am Kopf der zweiten Destillationskolonne gebildete Gemisch abführt, wobei man
c) die zweite Destillationskolonne bei einem höheren Druck als die erste Destillationskolonne betreibt und wobei man mindestens eines der beiden über Kopf der Destillationskolonnen abgezogenen Gemische einer Phasentrennung zuführt, wobei man im Fall der Phasentrennung (7) hinter der ersten Destillationskolonne lediglich die abgetrennte organische Phase der zweiten Destillationskolonne zuführt und im Fall einer Phasentrennung hinter der zweiten Destillationskolonne (16) ebenfalls eine Auftrennung in eine wäßrige und eine organische Phase erfolgt und wobei man einen Teilstrom vom Kopf oder Verstärkungsteil der zweiten Destillationskolonne in die erste Destillationskolonne zurückführt.

2. Verfahren nach Anspruch 1, dadurch gekennzeichnet, daß man der ersten Destillationskolonne eine Phasentrennung (7) nachschaltet.

3. Verfahren nach Anspruch 2, dadurch gekennzeichnet, daß man die in dem Phasentrenngefäß abgeschiedene, wäßrige Phase teilweise in die erste Kolonne zurückführt und den Rest ausschleust.

4. Verfahren nach Ansprüchen 1 bis 3, dadurch gekennzeichnet, daß man der zweiten Destillationskolonne im Verstärkungsteil einen Seitenstrom (13) entnimmt und diesen in die erste Destillationskolonne zurückführt.

5. Verfahren nach Anspruch 4, dadurch gekennzeichnet, daß man den Seitenstrom einem Phasentrenngefä,3 zuführt, die wäßrige Phase ausschleust und die organische Phase in die erste Destillationskolonne zurückführt.

## Claims

1. A process for separating butanol and dibutyl ether from a mixture containing water, dibutyl ether and n-butanol, 2-butanol or isobutanol, wherein
a) the mixture is introduced into a first distillation column (1), essentially butanol is separated off at the bottom of this distillation column and the mixture taken off at the top of the distillation column
b) is introduced into a second distillation column (12) and essentially dibutyl ether is separated off at the bottom of this second distillation column and the mixture formed at the top of the second distillation column is removed,
c) the second distillation column being operated at a higher pressure than the first distillation column and at least one of the two mixtures taken off via the top of the distillation columns being subjected to phase separation, only the organic phase separated off being fed to the second distillation column in the case of phase separation (7) downstream of the first distillation column, and separation into an aqueous and an organic phase also being effected in the case of phase separation downstream of the second distillation column (12), a part stream being recycled from the top or rectification section of the second distillation column to the first distillation column.

2. A process as claimed in claim 1, wherein a phase separation (7) is connected downstream of the first distillation column.

3. A process as claimed in claim 2, wherein some of the aqueous phase separated off in the phase separation vessel is recycled to the first column and the remainder is removed.

4. A process as claimed in any of claims 1 to 3, wherein a side stream (13) is removed from the rectification section of the second distillation column and is recycled to the first distillation column.

5. A process as claimed in claim 4, wherein the side stream is fed to a phase separation vessel, the aqueous phase is removed and the organic phase is recycled to the first distillation column.

## Revendications

1. Procédé pour séparer le butanol et l'éther dibutylique à partir d'un mélange contenant de l'eau, de l'éther dibutylique, du n-butanol, du 2-butanol et/ou de l'isobutanol, caractérisé par le fait que
a) on envoie le mélange à une première colonne à distiller (1) au pied de laquelle on sépare essentiellement le butanol et on envoie le mélange évacué en tête de la colonne à distiller
b) à une deuxième colonne à distiller (12) au pied de laquelle on sépare essentiellement l'éther dibutylique et on évacue le mélange formé en tête de cette deuxième colonne à distiller ;
c) on fait opérer la deuxième colonne à distiller à une pression supérieure à celle de la première colonne à distiller ; on envoie au moins un des deux mélanges évacués en tête des colonnes à distiller à une séparation de phases ; dans le cas de la séparation de phases (7) suivant la première colonne à distiller, on envoie simplement la phase organique séparée à la deuxième colonne à distiller ; et dans le cas d'une séparation de phases suivant la deuxième colonne à distiller (16) on sépare également une phase aqueuse et une phase organique et on recycle un courant partiel de la tête ou de la partie concentration de la deuxième colonne à distiller à la première colonne à distiller.

2. Procédé selon la revendication 1, caractérisé par le fait que la première colonne à distiller est suivie d'une séparation de phases (7).

3. Procédé selon la revendication 2, caractérisé par le fait que la phase aqueuse séparée dans le récipient à séparation de phases est recyclée en partie à la première colonne et le reste est évacué.

4. Procédé selon les revendications 1 à 3, caractérisé par le fait que, dans la partie concentration de la deuxième colonne à distiller, on prélève un courant latéral (13) qu'on recycle à la première colonne à distiller.

5. Procédé selon la revendication 4, caractérisé par le fait que l'on envoie le courant latéral à un récipient séparateur de phases, on évacue la phase aqueuse et on recycle la phase organique à la première colonne à distiller.
